# EUROPÄISCHE PATENTSCHRIFT

(11) **EP 3 330 270 B1**
(45) Veröffentlichungstag und Bekanntmachung des Hinweises auf die Patenterteilung: **05.04.2023**
(21) Anmeldenummer: 16201389.0
(22) Anmeldetag: 30.11.2016
(51) Int. Cl.: C07D 471/14, A61K 31/4745

(54) **PYRIDOPHENANTHROLINDERIVAT, DESSEN HERSTELLUNG UND VERWENDUNG ALS ARZNEIMITTEL**
PYRIDOPHENANTHROLINE DERIVATIVE, ITS PRODUCTION AND UTILISATION AS MEDICINE
DÉRIVÉ DE PYRIDOPHÉNANTHROLINE, SA FABRICATION ET SON UTILISATION EN TANT QUE MÉDICAMENT

(43) Veröffentlichungstag der Anmeldung: 06.06.2018
(73) Patentinhaber: Christian-Albrechts-Universität zu Kiel, 24118 Kiel (DE)
(72) Erfinder: Clement, Bernd, 24106 Kiel (DE); Meier, Christopher, 24118 Kiel (DE); Steinhauer, Tamara Natalie, 24105 Kiel (DE)
(74) Vertreter: Hamm&Wittkopp Patentanwälte PartmbB

(56) Entgegenhaltungen:
- WO-A1-2013/150140
- MEIER CHRISTOPHER ET AL: "Synthesis and physicochemical characterization of novel 6-aminopyrido[3,4-c][1,9]phenanthrolines as aza-analogs of benzo[c]phenanthridines", TETRAHEDRON, ELSEVIER SCIENCE PUBLISHERS, AMSTERDAM, NL, Bd. 68, Nr. 44, 4. November 2012 (2012-11-04), Seiten 9105-9112, XP002697380, ISSN: 0040-4020, DOI: 10.1016/J.TET.2012.08.047 [gefunden am 2012-08-28]
- LARS STENZEL: "Synthese neuer Benzo[c]phenanthridin-Derivate und deren Stickstoff-Analoga als potentielle Zytostatika", DISSERTATION ZUR ERLANGUNG DES DOKTORGRADES DER MATHEMATISCH- NATURWISSENSCHAFTLICHEN FAKULTÄT DER CHRISTIAN- ALBRECHTS- UNIVERSITÄT ZU KIEL , 9. November 2009 (2009-11-09), 2009, Seiten 1-136, XP002697378, Gefunden im Internet: URL:http://eldiss.uni-kiel.de/macau/receiv e/dissertation_diss_00004435 [gefunden am 2017-03-10]

## Beschreibung

Die Erfindung betrifft ein Pyridophenanthrolinderivat, dessen Herstellung und Verwendung in der Medizin insbesondere zur Therapie von Krebs.

Aus der WO 2013/150140 A1 sind Pyridophenanthrolinderivate und ihre Herstellung bekannt. In dieser Patentfamilie wird auch erstmals die pharmakologische, insbesondere die antitumorale, Wirkung von Pyridophenanthrolinderivaten beschrieben.

Ein erfolgversprechender Weg in der Therapie von Krebs ist die Hemmung der Topoisomerasen, von Enzymen, das für die Erzeugung transienter Einzel- und Doppelstrangbrüche zum Erhalt der Topologie der DNA während der Transkription und Replikation im Rahmen der DNA- und RNA-Synthese benötigt wird. Die Hemmung der Topoisomerase führt zum Absterben der Krebszellen. Es ist dabei in der therapeutischen Anwendung die Hemmung von zwei Typen von Topoisomerasen bekannt: Typ I und Typ II. [http://www.cancer.gov/publications/dictionaries/cancer-terms?CdrID=46665]

Als besonders vielversprechend in der Entwicklung neuer Hemmstoffe humaner Topoisomerasen in der Krebstherapie hat sich eine gleichzeitige Hemmung der beiden Enzymformen I und II herausgestellt. Ein entscheidender Vorteil besteht darin, dass einer Hochregulierung des jeweils eines Enzyms durch die selektive Hemmung des anderen, wie es bei aktuell in der Therapie eingesetzten Topoisomerasehemmem der Fall ist, durch die gleichmäßige Hemmung beider Formen I und II entgegengewirkt werden kann.

So diskutieren Schwandt et al. die gute Wirksamkeit dualer Topoisomerasehemmer bei der Therapie des kleinzelligen Lungenkarzinoms. [Schwandt et al.: "Brief Report: Phase II Trial of Rebeccamycin Analogue, a Dual Topoisomerase I and II Inhibitor, in Relapsed "Sensitive" SmallCell Lung Cancer" Thorac Oncol. 2012 April; 7(4): 751-754, doi:10.1097/JTO.0b013e31824abca2]

Van Gijn et al. beschreiben die Wirksamkeit einiger Topoisomerasehemmer gegen diverse Arten von Krebs, besonders vielversprechend zeigen sich einige als duale Topoisomerasehemmer wirksame Verbindungen. Das als dualer Topoisomerasehemmer wirksame "Intoplicin" zeigt eine hohe Aktivität gegen Mammakarzinome, Ovarialkarzinome, Colonkarzinome, Nierenzellkarzinome und Nicht-kleinzelligen Lungenkrebs. In klinischen Phase I erwies sich eine Hepatotoxizität der Substanz als doslimitierend, was für einige Patienten letale Konsequenzen hatte. Das ebenfalls als dualer Topoisomerasehemmer wirksame TAS-103 zeigt Wirksamkeit bei Kopf-Hals-Tumoren, Colonkarzinomen, Nierenkarzinomen und Nicht-kleinzelligem Lungenkrebs, die Wirksamkeit reicht aus um die Patienten über einen Zeitraum von einigen Monaten stabil zu halten eine Heilung konnte nicht erzielt werden, [van Gijn et al. "Dual topoisomerase inhibitors I/II" J. Oncol Pharm Practice, Voi 6, No 3(2000)].

Der duale Topoisomerasehemmer Pyrazoloacridin (PZA, NSC 366140) zeigt nach Adjei et al. Wirkung bei (metastasierenden) malignen Melanomen, Leberzellkarzinomen und Mesotheliomen. In der klinischen Studie konnte hier keine Heilung aber eine Stabilisierung der Patienten über einige Monate erreicht werden. [Adjei et al. "A phase I and pharmacologic study of pyrazoloacridine (NSC 366140) and carboplatin in patients with advanced cancer", Investigational New Drugs 20: 297-304, 2002.]

Nach Grem et al. kann Pyrazoloacridin (PZA) in Phase II der klinischen Studie bei Pankreaskarzinomen, Ovarialkarzinomen und Prostatakrebs in einigen Fällen zumindest zu einer partiellen Remission führen. [ Grem et al. " A Phase I Pharmacologic and Pharmacodynamic Study of Pyrazoloacridine Given as a Weekly 24-Hour Continuous Intravenous Infusion in Adult Cancer Patients" Clinical Cancer Research 2149 Vol. 8, 2149-2156, July 2002]

De Jonge et al. beschreiben die Wirksamkeit des dualen Topoisomerasehemmer XR11576 gegen Colonkarzinome, Ovarialkarzinome, Mammakarzinome und Lungenkarzinome. In in vivo Xenograft-Studien konnten hohe Aktivitäten gegen Colonkarzinome und das nichtkleinzellige Lungenkarzinom gefunden werden. In klinischen Phase I Studien konnte der duale Topoisomerasehemmer XR11576 bei maligne Melanomen, Zervixkarzinomen und Krebserkrankungen der Ohrspeicheldrüse Wirkung entfalten, es zeigte sich eine Stabilisierung allerdings kein Rückgang der Erkrankung. [de Jonge et al. "Phase I and pharmacokinetic study of XR11576, an oral topoisomerase I and II inhibitor, administered on days 1-5 ofa 3-weekly cycle in patients with advanced solid tumours" British Journal of Cancer (2004) 91, 1459 - 1465]

Trotz der vielversprechenden Ansätze kann noch kein dualer Hemmstoff für eine zytostatische Therapie eingesetzt werden, was einen enormen Bedarf an neuen Verbindungen aus dieser speziellen Wirkstoffklasse unterstreicht.

Es ist daher Aufgabe der Erfindung neue Arzneimittel zur Krebstherapie zur Verfügung zu stellen.

Des Weiteren ist es Aufgabe der Erfindung neue Verbindungen zur Hemmung der Topoisomerasen zur Verfügung zu stellen.

Darüber hinaus ist es Aufgabe der Erfindung neue Verbindungen zur gleichzeitigen Hemmung der Topoisomerase Typ I und Topoisomerase Typ II zur Verfügung zu stellen.

Ferner ist es Aufgabe der Erfindung ein Verfahren zur Herstellung der erfindungsgemäßen Verbindungen zur Verfügung zu stellen.

Die Aufgabe der Erfindung wird gelöst durch eine Verbindung der Formel 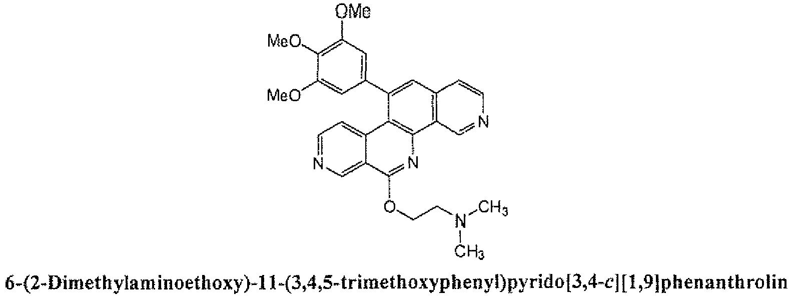 sowie deren Salze und/oder Solvate.

In einer besonderen Ausführungsform wird die Aufgabe gelöst durch das Salz der Verbindung 6-(2-Dimethylaminoethoxy)-11-(3,4,5-trimethoxyphenyl)pyrido[3,4-*c*][1,9]-phenanthrolinhydrochlorid als Prodrug für 6-(2-Dimethylaminoethoxy)-11-(3,4,5-trimethoxyphenyl)pyrido[3,4-c][1,9]phenanthrolin, sowie dessen Salze und Solvate können die dem Fachmann bekannten Prodrugkonzepte für Amine zum Einsatz kommen. Dabei sollte eine Substitution des tertiären Amins der Seitenkettenstruktur in Position 6 erfolgen. Die geeigneten Prodrugkonzepte für eine derartige Substitution des tertiären Amins der Seitenkettenstruktur in Position 6 finden sich insbesondere in [Simplicio et al., Prodrugs for Amines, Molecules, 2008, 13: 519-547].

Die Aufgabe der Erfindung wird ebenfalls gelöst durch ein Verfahren, dass die folgenden Schritte umfasst:

Herstellung einer Verbindung umfassend die Schritte
i. Umsetzung von substituierten Benzaldehyden mit 4-Methylpyridin-3-carbonitril in aprotischen, dipolaren Lösungsmitteln in Gegenwart von Basen
ii. Dehydrierung des Produktes aus i zu einem vollständig konjugierten System, unter Einführung einer Doppelbindung in 11,12-Position
iii. Diazotierung und anschließende Hydrolyse des semicyclischen Amidins in eine Laktam-Struktur
iv. Veretherung des Laktims im Sinne einer Mitsunobu-Reaktion

Das Reaktionsschema zeigt zur Erläuterung die Darstellung der Syntheseschritte in spezifischer, die Allgemeinheit nicht einschränkender Form:

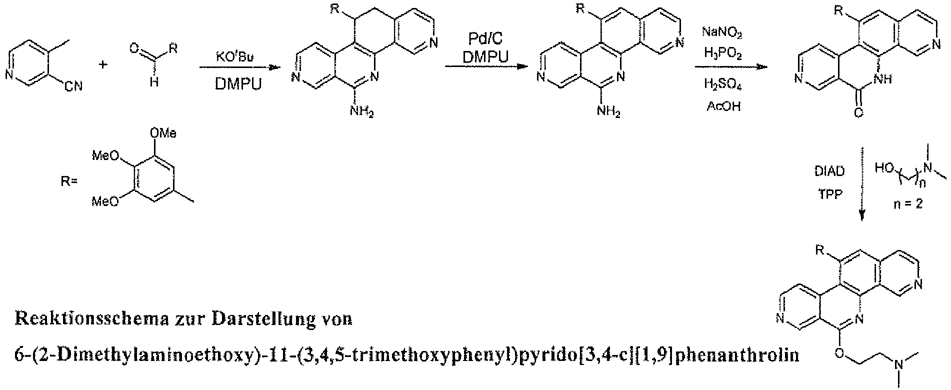

Die Erfindung betrifft auch Arzneimittel, welche das erfindungsgemäße Pyridophenanthrolinderivat enthalten. Diese Arzneimittel können dabei neben dem Phenanthrolinderivat dem Fachmann wohlbekannte pharmazeutische Hilfsstoffe enthalten wie:
1. Füllstoffe
2. Bindemittel
3. Fließregulierungsmittel
4. Sprengmittel
5. Schmiermittel
6. Magensaftresistente Überzüge und
7. Retardierungsmittel.

Hierzu lassen sich die erfindungsgemäß hergestellten Verbindungen gegebenenfalls in Kombination mit anderen Wirksubstanzen, zusammen mit einem oder mehreren inerten üblichen Trägerstoffen und/oder Verdünnungsmittel, z.B. mit Maisstärke, Milchzucker, Rohrzucker, mikrokristalliner Zellulose, Magnesiumstereat, Polyvinylpyrrolidon, Zitronensäure, Weinsäure, Wasser, Wasser/Ethanol, Wasser/ Glycerin, Wasser/Sorbit, Wasser/Polyethylglycol, Wasser/Propylenglycol, Cetylstearylalkohol, Carboxymethylcellulose oder fetthaltigen Substanzen wie Hartfett oder deren geeigneten Gemischen, in üblichen galenischen Zubereitungen wie Tabletten, Dragees, Kapseln, Pulver, Suspensionen, Zäpfchen oder injizierbaren Lösungen einarbeiten.

Die Erfindung betrifft auch die Verwendung der erfindungsgemäßen Verbindungen und Arzneimittel zur Behandlung von Leiden die durch Topoisomerasehemmer behandelt werden können. Insbesondere solche Leiden bei denen sich eine Behandlung mit dualen Topoisomerasehemmeren als vorteilhaft erwiesen hat.

Bei den erfindungsgemäß behandelbaren Leiden handelt es sich bevorzugt um Krebserkrankungen. Besonders bevorzugt sollen mit den erfindungsgemäßen Verbindungen und Arzneimitteln solche Krebserkrankungen behandelt werden, bei denen sich duale Topoisomerasehemmer bereits als wirksam erwiesen haben.

Besonders bevorzugt sind die erfindungsgemäß behandelbaren Krebserkrankungen ausgewählt aus der Gruppe der Mammakarzinome, Ovarialkarzinome, Colonkarzinome, Nierenkarzinome, Kopf-Hals-Tumore, kleinzelligen und nicht-kleinzelligen Lungenkarzinome, malignen Melanome, Leberzellkarzinome, Mesotheliome, Pankreaskarzinome, Prostatakrebs, Brustkarzinome, Zervixkarzinome und Krebserkrankungen der Ohrspeicheldrüse.

Im Folgenden soll die Allgemeinheit der Lehre nicht einschränkend die Herstellung und Wirksamkeit der erfindungsgemäßen Verbindung dargestellt werden.

### Beispiel 1 (erfindungsgemäß)

### Synthese von 6-(2-Dimethylaminoethoxy)-11-(3,4,5-trimethoxyphenyl)pyrido[3,4-c][1,9]-phenanthrolinhydrochlorid (Verbindung 1) (P8D 6)

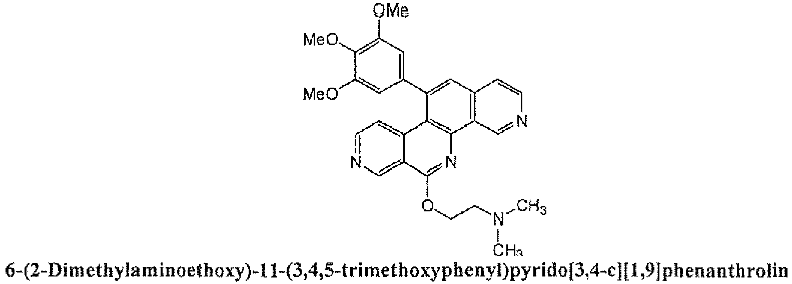

Die Darstellung von 6-(2-Dimethylaminoethoxy)-11-(3,4,5-trimethoxyphenyl)pyrido [3,4-c][1,9]phenanthrolinhydrochlorid (Beispiel 1) verläuft über 4 Stufen.

### Stufe 1: Synthese von 6-Amino-11-(3,4,5-trimethoxyphenyl)-11,12-dihydropyrido[3,4-c]-[1,9]phenanthrolin:

Eine Lösung aus 4-Methylpyridin-3-carbonitril (500 mg, 4.2 mmol) und 412 mg (2,1 mmol) 3,4,5-Trimethoxybenzaldehyd in 10 mL DMPU wird unter Stickstoffatmosphäre zu einer Lösung bestehend aus 600 mg (5,35 mmol) KOtBu in 5 mL DMPU getropft. Nach einer Reaktionszeit von 3 Stunden wird das Reaktionsgemisch auf 80 mL Eiswasser hydrolysiert. Der entstandene Niederschlag wird abgesaugt. Die Aufreinigung des Rohproduktes geschieht flashchromatographisch an Kieselgel (Fließmittel: Dichlormethan/Methanol (5-20%)). Der erhaltene Feststoff wird 24 h im Ölpumpenvakuum getrocknet. Ausbeute: 244 mg (28%).

### Stufe 2: Synthese von 6-Amino-11-(3,4,5-trimethoxyphenyl)pyrido[3,4-c][1,9]phenanthrolin:

200 mg (0,48 mmol) 6-Amino-11-(3,4,5-trimethoxyphenyl)-11,12-dihydropyrido[3,4-c]-[1,9]phenanthrolin werden in 5 - 10 mL DMPU gelöst und Palladium (10%) auf Aktivkohle (30 Gewichtsprozent) wird hinzugefügt. Die Suspension wird 10 min im Stickstoffstrom zum Rückfluss erhitzt. Nach dem Erkalten wird der Katalysator abfiltriert und mit 50 mL Dichlormethan nachgewaschen. Das Filtrat wird am Rotationsverdampfer eingeengt und die verbleibende Lösung auf 80 mL Eiswasser gegeben, entstehender Niederschlag wird abfiltriert. Entsteht kein Niederschlag wird mit konzentrierter Salzsäure auf pH 2 eingestellt und das DMPU mit 3 x 50 mL Dichlormethan aus der wässrigen Phase extrahiert. Im Anschluss wird die wässrige Phase mit 25-%iger wässriger

Ammoniaklösung auf pH 9 gebracht und der entstandene Niederschlag abfiltriert. Die Aufreinigung des Rohproduktes geschieht mittels Flashchromatographie an Kieselgel (Fließmittel: Dichlormethan/Methanol (10%)). Der erhaltene Feststoff wird 24 h im Ölpumpenvakuum getrocknet. Ausbeute: 149 mg (75%).

### Stufe 3: Synthese von 11-(3,4,5-Trimethoxyphenyl)-5,6-dihydropyrido[3,4-c][1,9]phenanthrolin-6-on:

3 N Schwefelsäure (5 mL) wird auf 0°C abgekühlt und langsam portionsweise nacheinander NaNO₂ (250 mg, 36 mmol) und auf 4°C vorgekühlte Phosphinsäure (1 mL, 1,2 mmol, 50 Gewichtsprozent in H₂O) dazugegeben. Eine zweite Lösung bestehend aus 300 mg (6-Amino-11-(3,4,5-trimethoxyphenyl)pyrido[3,4-c][1,9]phenanthrolin in 5 mL Eisessig und 2 mL Wasser wird langsam zu der ersten Lösung getropft. Nach Zugabe einer Spatelspitze Kupfer(II)sulfat wird das Lösungsgemisch eine Stunde bei 0°C gerührt und im Anschluss 24 Stunden im Kühlschrank bei 4-7°C gelagert. Danach wird die Lösung filtriert und das Filtrat mit 25%iger wässriger Ammoniaklösung auf pH 9 eingestellt, bis eine tiefblaue Färbung entsteht. Das hierbei entstehende Präzipitat wird abgesaugt, mit Wasser gewaschen (3 x 50 mL) und im Vakuum getrocknet. Die Aufreinigung des Rohproduktes erfolgt mittels Flashchromatographie an Kieselgel (Fließmittel: Dichlormethan/Methanol (5-20%)). Der erhaltene Feststoff wird 24 Stunden im Ölpumpenvakuum getrocknet. Ausbeute: 243 mg (81 %),

### Stufe 4: Synthese von 6-(2-Dimethylaminoethoxy)-11-(3,4,5-trimethoxyphenyl)pyrido[3,4-c]-[1,9]phenanthrolinhydrochlorid:

11-(3,4,5-Trimethoxyphenyl)-5,6-dihydropyrido[3,4-c][1,9]phenanthrolin-6-on (300 mg, 0,73 mmol) wird mit Triphenylphosphin (571 mg, 2,2 mmol) in 50 mL frisch getrocknetem THF gelöst. Anschließend werden nacheinander 2-Dimethylaminoethanol (196 mg, 2,2 mmol) und DIAD (445 mg, 2,2 mmol) hinzugetropft. Die Suspension wird unter Ausschluss von Wasser 72 Stunden bei Raumtemperatur gerührt, wobei sich nach und nach eine klare Lösung ergibt. Nach Beendigung der Reaktion wird das Lösungsmittel am Rotationsverdampfer entfernt und der erhaltene Feststoff im Vakuum getrocknet. Die Aufreinigung des Rohproduktes erfolgt per Flashchromatographie an Kieselgel (Fließmittel: Dichlormethan/Methanol (10-30%)) und das resultierende Produkt wird im Anschluss 24 Stunden im Ölpumpenvakuum getrocknet. Das Produkt wird in trockenem Dichlormethan gelöst und für 5 min mit HCl(g) begast. Das Lösungsmittel wird am Rotationsverdampfer entfernt und der erhaltene Feststoff 24 Stunden im Ölpumpenvakuum getrocknet.

### C₂₈H₂₉ClN₄O₄ (521.01)

| | |
|---|---|
| Ausbeute: | 274 mg (72%, gefällt als Hydrochlorid nach flashchromato-graphischer Aufreinigung, Kieselgel, CH₂Cl₂/MeOH (10-30%)) eines gelben Feststoffs |
| Schmelzpunkt: | 206°C |

### ¹H-NMR (300 MHz, DMSO-d6):

δ/ppm = 2.96 (d, 6H, RHN(CH₃)₂), 3.74 (s, 6H, 2 x OCH₃), 3.81 (s, 5H, OCH₃, H-2'), 5.33 (m, 2H, H-1'), 6.88 (s, 2H, H-2", H-6"), 7.37 (dd, 1H, H-10, ³J = 6.2 Hz, ⁴J = 0.8 Hz), 8.28 (s, 1H, H-12), 8.56 (d, 1H, H-1, ³J = 6.3 Hz), 8.74 (d, 1H, H-9, ³J = 6.2 Hz), 8.95 (d, 1H, H-2, ³J = 6.3 Hz), 10.08 (d, 1H, H-7, J = 0.7 Hz), 10.64 (s, 1H, H-4), 11.25 (m, 1H, RHN(CH₃)₂).

Das Signal der Methylenprotonen an C-2' wird durch das Singulett der Methoxygruppe bei 3.81 überlagert.

### ¹³C-NMR (75 MHz, DMSO-d6):

δ/ppm = 42.5 (2C, RHN(CH₃)₂), 54.8 (C-2`), 56.2 (2C, 2 x OCH₃), 60.4 (OCH₃), 61.8 (C-1'), 106.0 (2C), 115.3,118.0, 119.2, 124.0, 124.1, 126.0,136.1,136.5, 138.2, 139.0, 139.6, 143.8, 144.3, 147.1, 148.9, 153.7 (2C), 159.6.

### IR (ATR):

cm⁻¹ =3399, 2946, 2467, 1639, 1606, 1578, 1453, 1412, 1338, 1111, 988,

### MS (ESI):

m/z = 485 ([M+H]⁺, 41%), 414 ([M-C4H9N+H]⁺, 100%), 243 ([M+2H]⁺⁺, 44%).

### Elementaranalyse (C₂₈H₂₈N₄O₄ · 3 HCl):

| | | | |
|---|---|---|---|
| Berechnet: | C 56.62 | H 5.26 | N 9.43, |
| gefunden: | C 56.20 | H 5.52 | N 9.40. |

### Beispiel 2 (nicht erfindungsgemäß)

### Synthese von 6-(3-Dimethylaminopropoxy)-11-(3,4,5-trimethoxyphenyl)pyrido[3,4-c][1,9]-phenanthrolin (Verbindung 2)

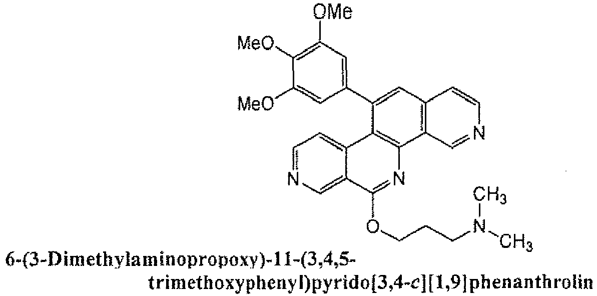

Die Darstellung von 6-(3-Dimethylaminopropoxy)-11-(3,4,5-trimethoxyphenyl)pyrido[3,4-*c*][1,9]phenanthrolin (Beispiel 2) verläuft ebenfalls über 4 Stufen.

Stufen 1 bis 3 verlaufen analog der Darstellung von Beispiel 1.

Stufe 4: 11-(3,4,5-Trimethoxyphenyl)-5,6-dihydropyrido[3,4-*c*][1,9]phenanthrolin-6-on (300 mg, 0,73 mmol) wird mit Triphenylphosphin (571 mg, 2,2 mmol) in 50 mL frisch getrocknetem THF gelost. Anschließend werden nacheinander 227 mg (2,2 mmol) Dimethylaminopropanol und DIAD (445 mg, 2,2 mmol) hinzugetropft.

Die Suspension wird unter Ausschluss von Wasser 72 Stunden bei Raumtemperatur gerührt, wobei sich nach und nach eine klare Lösung ergibt. Nach Beendigung der Reaktion wird das Lösungsmittel am Rotationsverdampfer entfernt und der erhaltene Feststoff im Vakuum getrocknet. Die Aufreinigung des Rohproduktes erfolgt per Flashchromatographie an Kieselgel (Fließmittel: Dichlormethan/Methanol (10-30%)) und das resultierende Produkt wird im Anschluss 24 Stunden im Ölpumpenvakuum getrocknet.

### C₂₉H₃₀N₄O₄ (498.57)

| | |
|---|---|
| Ausbeute: | 163 mg (55%, Flashchromatographie, Kieselgel, CH₂Cl₂MeOH (10-30%)) eines gelben Feststoffs |
| Schmelzpunkt: | 172°C |

### ¹H-NMR 300 MHz, DMSO-d₆ + 1% PCI (36%) in D₂O):

δ/ppm = 2.46 (m, 2H, H-2`), 2.83 (s, 6H, RHN(CH₃)₂), 3.49 (m, 2H, H-3'), 3.72 (s, 6H, 2 x OCH₃), 3.78 (s, 3H, OCH₃), 5.07 (t, 2H, H-1'), 6.90 (s, 2H, H-2", H-6"), 7.71 (dd, 1H, H-10, *³J =* 6.6 Hz), 8.37 (s, **1H,** H-12), 8.66 (d, 1H, H-1, ³*J* = 6.5 Hz), 8.83 (d, 1H, H-9, ³*J* = 6.6 Hz), 8.96 (d, 1H, H-2, *³J* = 6.4 Hz), 10.10 (s, 1H, H-7, *J* = 0.7 Hz), 10.64 (s, 1H, H-4).

### ¹³C-NMR (75 MHz, DMSO-d₆ + 1% DCl (36%) in D₂O):

δ/ppm = 23.3 (C-2'), 42.2 (2C, RHN(CH₃)₂), 53.9 (C-3'), 56.5 (2C, 2 x OCH₃), 60.7 (OCH₃), 66.0 (C-1'), 106.4 (2C), 116.9, 117.6, 122.6, 124.2, 125.0, 126.6, 135.5, 135.7, 138.7, 139.5, 140.6, 143.4, 144.1, 144.5, 146.4, 148.1,154.1 (2C), 160.4.

### IR (ATR):

cm⁻¹ = 3363, 2936, 2834, 1610, 1566, 1407, 1343, 1230, 1126, 1112, 830.

### MS (ESI):

m/z = 499 ([M+H]⁺, 51%), 414 ([M-C₅H₁₁N+H]⁺, 100%), 250 ([M+2H]⁺⁺, 73%).

### HRMS (ESI):

m/z berechnet für [M+H]⁺: 499.2345,
m/z gefunden für [M+H]⁺: 499.2326.

### Beispiel 3 (nicht erfindungsgemäß)

### Synthese von 11-(3-Chlorphenyl)-6-(2-dimethylaminoethoxy)-11,12-dihydropyrido[3,4-c]-[1,9]phenanthrolinhydrochlorid (Verbindung 3) (P16 6)

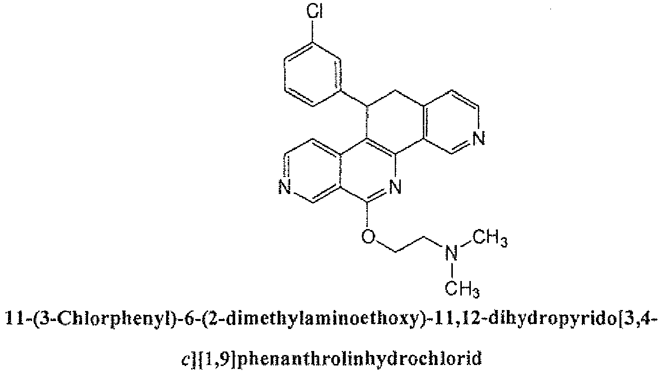

Die Darstellung von 11-(3-Chlorphenyl)-6-(2-dimethylaminoethoxy)-11,12-dihydro-pyrido[3,4-*c*][1,9]phenanthrolinhydrochlorid (Beispiel 3) verläuft über 3 Stufen. Stufe 1: Synthese von 6-Amino-11-(3-Chlorphenyl)-11,12-dihydropyrido[3,4-*c*][1,9]phenanthrolin: 3-Chlorbenzaldehyd (295 mg, 2,1 mmol) wird analog der Vorschrift aus Stufe 1 für Beispiel 1 umgesetzt. Ausbeute: 241 mg (32%).

Stufe 2: Synthese von 11-(3-Chlorphenyl)-5,6,11,12-tetrahydropyrido[3,4-*c*][1,9]phenanthrolin-6-on: 300 mg (0,84 mmol) 6-Amino-11-(3-Chlorphenyl)-11,12-dihydropyrido[3,4-*c*][1,9]phenanthrolin werden analog der Vorschrift aus Stufe 3 für Beispiel 1 umgesetzt. Ausbeute: 277 mg (91%).

Stufe 3: 300 mg (0,83 mmol) 11-(3-Chlorphenyl)-5,6,11,12-tetrahydropyrido[3,4-*c*][1,9]-phenanthrolin-6-on werden analog der Vorschrift aus Stufe 4 für Beispiel 1 umgesetzt.

### C₂₅H₂₄Cl₂N₄O (467.39)

| | |
|---|---|
| Ausbeute: | 177 mg (45%, als Hydrochlorid gefällt nach flashchromato-graphischer Aufreinigung, Kieselgel, CH₂Cl₂MeOH (10-30%)) eines gelben Feststoffs |
| Schmelzpunkt: | 188°C |

### ¹H-NMR (300 MHz, DMSO-d₆):

δ/ppm = 2.93 (t, 6H, RHN(CH₃)₂), 3.62 (d, 1H, H-12a, ²*J*_{H-12a/H-12b} = 17.2 Hz), 3.75 (q, 2H, H-2', ³*J* = 5.1 Hz), 3.84 (dd, 1H, H-12-b, ²*J*_{H-12b/H-12a} = 17.3 Hz, ³*J*_{H-12b/H-11} = 7.9 Hz), 5.15 (m, 2H, H-1`), 5.32 (d, 1H, H-11, ³*J*_{H11/H12-b}= 7.8 Hz), 6.80-6.86 (m, 1H, H-6", ³*J* = 7.7 Hz), 7.14 (m, 1H, H-5",³*J* = 7.8 Hz), 7.18-7.23 (m, 1H, H-4",³*J* = 7.8 Hz), 7.30 (m, 1H, H-2"), 7.94 (d, 1H, H-1, ³*J* = 5.7 Hz), 8.05 (d, 1H, H-10, ³*J* = 6.4 Hz), 8.80 (d, 1H, H-2, *³J =* 5.7 Hz), 8.85 (d, 1H, H-9, ³*J* = 6.3 Hz), 9.60 (s, 1H, H-4), 10.09 (s, 1H-H-7), 11.21 (m, 1H, RHN(CH₃)₂).

### ¹³C-NMR (75 MHz DMSO-d₆):

δ/ppm = 35.0 (C-12), 35.2 (C-11), 42.3, 42.4 (2C, RHN(CH₃)₂), 54.7 (C-2'), 61.4 (C-1'), 114.8, 117.6, 120.8, 125.7, 126.4, 127.1, 127.3, 130.5, 131.8, 133.4, 138.5, 140.9, 142.9, 134.3, 143.7, 145.5, 148.4, 160.0.

### IR (ATR):

cm⁻¹= 3364, 2970, 2468, 1632, 1592, 1561, 1465, 1425, 1342, 829.

### MS (ESI):

m/z = 433 ([M+H]⁺, ³⁷Cl, 18%), 431 ([M+H]⁺, ³⁵Cl, 44%), 362 ([M-C₄H₉N+H]⁺, ³⁷Cl, 35%), 360 ([M-C₄H₁₀N+H]⁺, ³⁵Cl, 100%), 343 (3%), 248 ([M-3-Chlorbenzen+H]⁺, 6%).

### HRMS (ESI):

m/z berechnet für [M+H]⁺: 431.1633,
m/z gefunden für [M+H]⁺: 431.1613.

### Pharmakologische Testergebnisse

Zur Untersuchung der pharmakologischen Eigenschaften wurden die Verbindungen Beispiel 1 bis 3 (wobei nur Beispiel 1 erfindungsgemäß ist) im "DTP NCI-60 in vitro human tumor cell line screening" des National Cancer Institute (NCI), Bethesda, Maryland, USA, untersucht. Getestet wurde gegen 60 verschiedene humanpathogene Tumorzelllinien, die neun Krebsarten entstammten (Leukämie, nichtkleinzelliges Lungenkarzinom, Dickdarmkrebs, ZNS-Krebs, Melanom, Eierstockkrebs, Nierenkrebs, Prostatakrebs, Brustkrebs). Zur Ermittlung der Wirksamkeit werden die Tumorzellen den Verbindungen über zwei Tage ausgesetzt und danach indirekt über die Bestimmung der Proteinbiomasse mit Sulforhodamin B (SRB) die Wachstumshemmung ermittelt. Unbehandelte Kulturen dienen als Referenz. Der "meangraph midpoint" (MG_MID_{GI50}) entspricht einer mittleren Ansprechrate aller getesteten humanen Zelllinien auf die getestete Substanz. Entlogarithmiert ergibt sich daraus die Konzentration die zu einer 50-prozentigen Wachstumshemmung aller Zelllinien bezogen auf unbehandelte Referenzzelllinien führt (GI_{50-NCI60}). Tabelle 1 zeigt sehr starke wachstumshemmende Wirkungen der drei Verbindungen 1 bis 3 in nanomolaren Konzentrationen im Mittel auf die 60 humanen Tumorzelllinien.

Die Wirksamkeit der Beispielverbindungen 1 bis 3 übertrifft teilweise deutlich die wachstumshemmenden Eigenschaften von in der Krebstherapie zugelassenen Arzneistoffen und Modellverbindungen aus der Klasse der Topoisomerase-Hemmstoffe, welches ebenfalls dasselbe Testsystem durchlaufen haben.

Aus der Klasse dualer Topoisomerase I/II-Hemmstoffe sind die Verbindungen Pyrazoloacridin (PZA) und Intoplicin aufgeführt, welche sich bereits in der klinischen Entwicklung befanden. Insbesondere gegenüber den beiden bekannten Topoisomerase TIII-Hemmern Pyrazoloacridin (PZA) und Intoplicin zeigen die Verbindungen aus Beispiel 1 und Beispiel 2 eine deutlich bessere antitumorale Wirkung.

**Tabelle 1: Mittlere Wachstumshemmung auf alle 60 Tumorzelllinien des DTP NCI-60 in vitro human tumor cell line screening**

| **Substanz** | **GI_{50/NCI-60} [µM]** |
|---|---|
| Beispiel 1 | **0,049** |
| Beispiel 2 | **0,052** |
| Beispiel 3 | **0,447** |

| **Topo I-Inhibitoren** | |
|---|---|
| *Camptothecin* | 0,044 |
| *Topotecan* | 0,014 |
| *Irinotecan* | 0,16 |

| **Topo II-Inhibitoren** | |
|---|---|
| *Etoposide* | 0,99 |
| *Teniposide* | 0,39 |

| **Duale Topo IIII-Inhibitoren** | |
|---|---|
| *PZA* | 0,20 |
| *Intoplicin* | 0,53 |

Zur Untersuchung der pharmakologischen Eigenschaften wurden die Verbindungen 1 bis 3 ebenfalls auf ihre Fähigkeit, die Aktivitäten humaner Topoisomerasen I, IIα, und IIβ zu hemmen, untersucht. Die enstsprechenden Modell-Topoisomerase-Hemmstoffe Camptothecin (Topoisomerase I) und Etoposid (Topoisomerase II) wurden als Referenzverbindungen mitgetestet. Tabelle 2 zeigt die Ergebnisse.

**Tabelle 2: Hemmung humaner Topoisomerasen I, IIα, und IIβ durch Beispiel 1:**

| **Substanz** | **GI₅₀ (Topoisomerase I)¹** | **GI₅₀ (Topoisomerase IIα)²** | **GI₅₀ (Topoisomerase IIβ)²** |
|---|---|---|---|
| Beispiel 1 | +++ | +++ | +++ |
| *Camptothecin* | + | n.b. | n.b. |
| *Etoposid* | n.b. | + | + |

| | | | |
|---|---|---|---|
| +++*:**IC₅₀*** = ***10-50 µM,* +*: IC₅₀*= *50-100 µM. ¹Daten aus Relaxationsassay; ²Daten aus*** ***Dekatenierungsassay; n.b.* = *nicht bestimmt.*** | | | |

Beispielverbindung 1 (Bsp. 1) zeigt in diesen Tests eine außerordentlich starke und gleichmäßige Inhibition der Aktivität aller humanen Topoisomerasen (IC₅₀ < 10 µM für alle drei getesteten Enzyme), die weitaus stärker als die Aktivitäten der Modell-Topoisomerase-Hemmer Etoposid und Camptothecin war. Überraschenderweise konnte nur Beispielverbindung 1 diese Eigenschaften eines potenten dualen Topoisomerase I/II Hemmstoffs aufweisen.

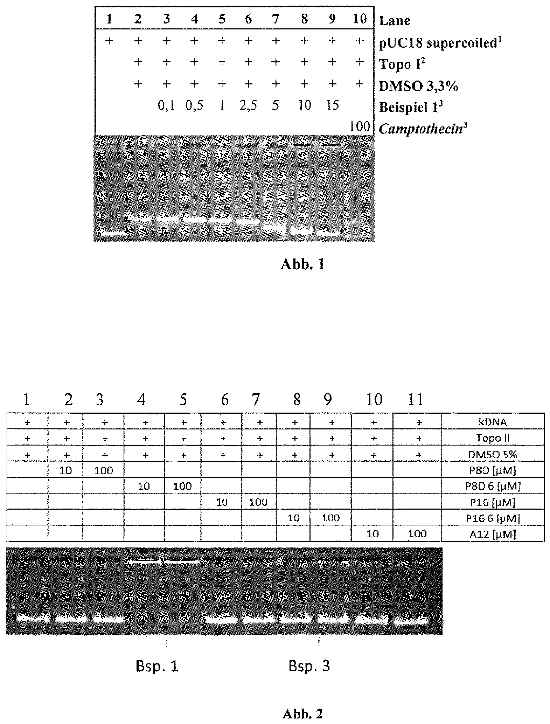

Abbildung 1 zeigt die Beeinflussung der Topoisomerase I Aktivität mit Hilfe des Relaxationsassays. Mit dem Relaxationsassay lässt sich die Inhibition humaner DNA-Topoisomerase I durch eine Substanz bestimmen. Topo 1 ist in der Lage, das superspiralisierte Plasmid pUC18 in die relaxierte Form zu überführen. Die resultierenden unterschiedlichen Wanderungseigenschaften der Plasmid-Isoformen in einem Agarose-Gel bei Anlegen eines elektrischen Feldes geben ein Maß für die Aktivität des Enzyms (Nitiss et al., Topoisomerase Assays, in: Enna et al., Curr. Prot. Pharmacol., 2012). Ersichtlich ist eine konzentrationsabhängige Hemmung der Topoisomerase I Aktivität durch steigende Konzentrationen der Beispielverbindung 1 (Lane 3 bis 9), die bei 10 µM nahezu vollständig ist, vgl. mit Lane 1, welche das überspiralisierte Plasmid ohne Topoisomerase I enthält. Lane 2 stellt die Negativkontrolle ohne Hemmstoff dar. Als Positivkontrolle diente Camptothecin in der Konzentration von 100 µM (Lane 10).

Abbildung 2 zeigt beispielhaft für die Hemmung der Topoisomerase IIα-Aktivität den Einfluss verschiedener Pyridophenanthroline (P8D, P16, P16 6 (entspricht Verbindung 3 aus Beispiel 3) und P8D 6 (entspricht Verbindung 1 aus Beispiel 1) und des Pyridoazacarbazols A12, auf das Enzym.

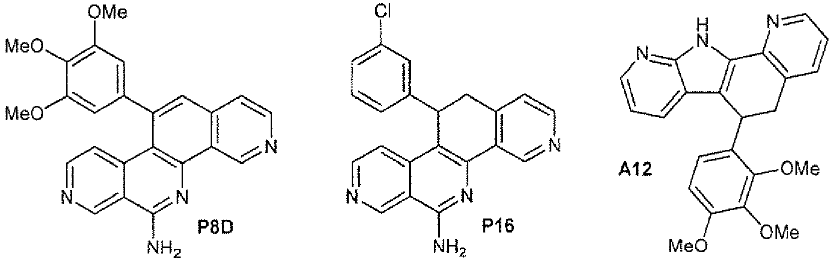

Verwendet wurde ein Dekatenierungsassay, mit dessen Hilfe sich die Inhibition humaner Topoisomerasen II (α/β) durch eine Substanz bestimmen lässt. Das Protein ist in der Lage, Kinetoplasten-DNA (kDNA), die in Form ineinander verzahnter Minizirkel (sog. Katenate) vorliegt, zu dekatenieren, d.h. in einzelne Minizirkel zu spalten. Diese sind nun im Unterschied zur ursprünglichen kDNA im Stande, in einem Agarosegel bei Anlegen eines elektrischen Feldes zu wandern (Nitiss et al., Topoisomerase Assays, in: Enna et al., Curr. Prot. Pharmacol., 2012.

In Abbildung 2 ist gezeigt, wie ausschließlich Beispielverbindung 1 (P8D 6) die Aktivität der Topo IIα potent zu hemmen vermochte, so dass es zu keiner Bildung entzahnter DNA-Minizirkel kam, ersichtlich an der Immobilität der Banden in den Lanes 4 und 5, verglichen mit Lane 1, die als Referenz ohne Hemmsubstanz mit aufgetragen wurde.

Lediglich Beispiel 1 konnte wider Erwarten sowohl in niedrigen (10 µM) als auch im hohen (100 µM) Konzentrationsbereich eine beispielhafte Hemmung der Topo IIα erzielen.

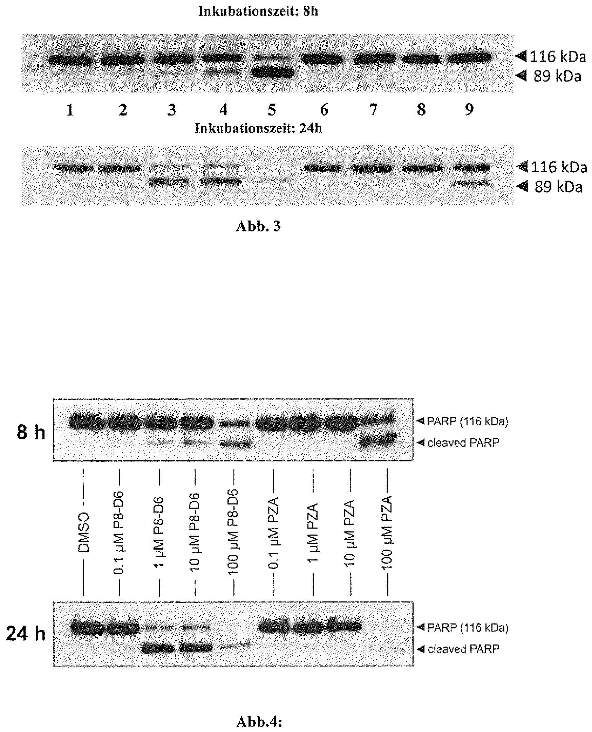

Für Beispielverbindung 1 wurde darüber hinaus Untersuchungen auf apoptotische Aktivität durchgeführt. Zur Ermittlung des apoptotischen Status einer Zelle kann das Enzym-PARP (Poly-ADP Ribose Polymerase) herangezogen werden, das beim Übergang der Zelle in die Apoptose in ein 89kDa Fragment gespalten wird, und somit als Apoptose-Marker dienen kann (KAUFMANN SH et al., Cancer Research, 1993; 53:3976-3985). In Abbildung 3 ist der Effekt verschiedener Konzentrationen von Beispiel 1 auf HeLa-Zellen im PARP-Assay dargestellt. Als Referenzverbindung wurde Etoposid gewählt und mitgetestet.

Abbildung 3 zeigt ein sehr schnelles Auftreten des apoptotischen Status der Zelle (89kDa Bande - gespaltenes PARP bereits bei 1 µM Konzentration an Beispielverbindung 1 sichtbar, keine Einleitung der Apoptose bei Etoposid ≤ 100 µM nach 8h). Nach 24h genügt eine 10⁻³-10⁻²-fach geringere Konzentration an Beispielverbindung 1 als an Etoposid, um das PARP-Spaltprodukt nachzuweisen. Beispielverbindung 1 hat somit eine herausragende apoptotische Wirkung.

Ein weiterer PARP-Assay wurde durchgeführt, um einen direkten Vergleich der proapoptotischen Wirkungen von Beispiel. 1 (hier: P8D 6) und dem potenten und literaturbekannten dualen Topoisomerase I/II-Hemmstoff Pyrazoloacridin (PZA) zu erhalten (Abb. 4).

Auch hier wird die außerordentlich starke pro-apoptotische Wirkung der Beispielverbindung 1 ersichtlich, während der duale Topoisomerasehemmer PZA erst in hohen Konzentrationen (100 µM) seine Wirkung entfaltet.

### Physikochemische Eigenschaften:

Neben den ausgezeichneten pharmakologischen Eigenschaften zeigen die Verbindungen physikochemische Vorteile, die deren hervorragende Eignung als Arzneistoffe weiter untermauern (Tabelle 3):

**Tabelle 3: Physikochemische Eigenschaften der Beispielverbindungen 1-3 (wobei nur Beispiel 1 erfindungsgemäß ist)**

| **Substanz** | **Löslichkeit pH 7,4 [mM]¹** | **H-Donatoren²** | **H-Akzeptoren** | **logD¹/ClogP³** | **Molekular-Molekular-gewicht [g/mol]** |
|---|---|---|---|---|---|
| Beispiel 1 | 0,87 ± 0,03 | 1 | 8 | 3,70/4,08 | 485 |
| Beispiel 2 | n.b. | 1 | 8 | 3,60/4,43 | 499 |
| Beispiel 3 | 5,28 ± 0,09 | 1 | 5 | 3,60/5,13 | 431 |

***Tabelle 3: Physikochemische Eigenschaften der Beispielverbindungen 1-3; ¹Bestimmt mittels HPLC-Analytik; ²Die tertiäre Aminstruktur wurde bei pH 7,4 als protoniert angesehen; ³Berechnet mit ChemDraw Ultra 12.0.***

Die Löslichkeit im millimolaren Bereich sowie die Anwendbarkeit der "rule of five" (nach: LIPINSKI CA et al., Advanced Drug Delivery Reviews, 2001; 46: 3-26) prognostizieren den Beispielverbindungen 1 bis 3 eine angemessene orale Bioverfügbarkeit, die somit neben einer für Zytostatika üblichen parenteralen Applikation auch eine perorale Gabe der Verbindungen erlaubt.

### Toxikologische Eigenschaften:

### Tolerierbarkeit von Beispielverbindung 1 (hier P8-D6) an weiblichen athymischen Nacktmäusen

### Studiendesign

Interne Studiennummer: P14.0073. Der Tierversuch, der unter der internen Studiennummer P14.0073 durchgeführt wurde, war durch die kommunale Regierungsbehörde und der zugehörigen Ethikkommission bewilligt (Regierungspräsidium Karlsruhe, 35-9185.81/G-24/11).

Im Rahmen einer *in vivo* Studie zur Bestimmung der Tolerierbarkeit von P8-D6 in weiblichen athymischen Nackmäusen wurde die Maximum Tolerated Dose (MTD) ermittelt.

Insgesamt wurden 18 weibliche athymische Nacktmäuse in einem Alter von 7 Wochen (Gruppe 1 und 2), 8 Wochen (Gruppe 3 und 4) und 9 Wochen (Gruppe 5 und 6) für die *in vivo* Studie herangezogen.

Um die Tolerierbarkeit von P8-D6 bei einer einmaligen intravenösen Applikation in die Schwanzvene zu ermitteln, erhielten die Studientiere der Gruppen 2, 4 und 6 (3 Tiere je Gruppe) die Verbindung P8-D6 in Dosen von 1 mg/kg Körpergewicht, 5 mg/kg Körpergewicht und 10 mg/kg Körpergewicht an den Studientagen 0, 7 beziehungsweise 14. Den Kontrolltieren der Gruppen 1,3 und 5 (3 Tiere je Gruppe) wurde PBS (Phosphate Buffered Saline) (pH 7,4) in einer Dosis von 10 mL/kg Körpergewicht an den jeweils selben Tagen appliziert. Die Studientiere wurden in einem Zeitraum bis zu 14 Tagen hinsichtlich klinischer Zeichen, Verhaltensänderungen und Überleben beobachtet. Die Bestimmung des Körpergewichtes erfolgte dreimal wöchentlich. Begonnen wurde am Tag der Applikation. Die Kriterien für einen frühzeitigen Abbruch der Studie beliefen sich aus ethischen Gründen auf eine Abnahme des Körpergewichtes um mehr als 20% vom Ausgangsgewicht. Während der Studie verstorbene und alle weiteren Studientiere wurden am Ende der Studienphase einer Nekropie unterzogen. Dabei wurden Organproben von sechs Studientieren entnommen (4 Tiere aus Gruppe 4, 1 Tier aus Gruppe 1, 1 Tier aus Gruppe 2 und ein Tier aus Gruppe 6).

### Ergebnisse

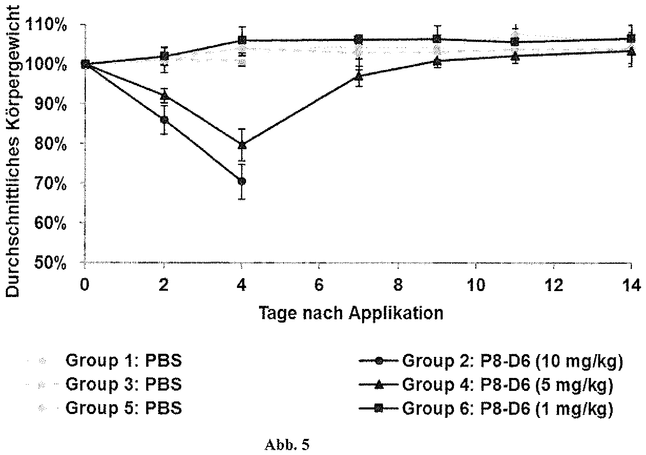

Die Abbildung 5 zeigt die Ergebnisse nach einmaliger intravenöser Applikation von P8D 6 in weibliche athymischen Nacktmäuse und intravenöse Gabe von PBS als Vergleich.

Die MTD Studie zeigte eine gute Tolerierbarkeit von P8-D6 in weiblichen athymischen Nacktmäusen bei einer Dosierung von 1 mg/kg Körpergewicht. Ferner konnte über die klinischen Zeichen und die Beobachtung des Körpergewichtes eine dosisabhängige Toxizität bei höheren Konzentrationen ermittelt werden, wobei der Verlust des Körpergewichtes und eine auftretende Teilnahmslosigkeit der Studientiere bei einer Dosis von 5 mg/kg Körpergewicht reversibel waren (Abb.5). Es zeigten sich außerdem bei keinem der Studientiere akute letale Begleiterscheinungen.

### Verzeichnis der Abbildungen:

Abbildung 1: Relaxationsassay auf Beeinflussung der Topoisomerase I-Aktivität: ¹Superspiralisiertes Plasmid pUC18, ²Topoisomerase I, ³Testkonzentrationen an Beispielverbindung 1. Einheit: µM.
Abbildung 2: Dekatenierungssassay auf Beeinflussung der Topoisomerase IIα-Aktivität: Lane 1 Referenz ohne Hemmsubstanz, Lane 2 und 3 (P8D), Lane 6 und 7 (P16), Lane 8 und 9 (P16 6) und Lane 10 und 11 (A12) sind Vergleiche mit ähnlichen Strukturen. Lane 4 und 5 ist Beispielverbindung 1, Lane 8 und 9 ist Beispielverbindung 3. kDNA = Kinetoplasten-DNA; Topo II = Topoisomerase IIα
Abbildung 3: PARP-Apoptose Assay (HeLa-Zellen).1: DMSO-Kontrolle (kein Inhibitor), 2-5: Beispiel 1 (0,1; 1; 10; 100 µM); 6-9: Etoposid (0,1; 1; 10; 100 µM).
Abbildung 4: PARP-Assay zum Vergleich von Beispiel 1 und dem dualen Topoisomerase I/II-Hemmstoff Pyrazoloacridin (PZA)
Abbildung 5: Einmalige intravenöse Applikation von Beispielverbindung 1 (P8-D6) in weibliche athymischen Nacktmäuse und intravenöse Gabe von PBS als Vergleich

## Patentansprüche

1. Verbindung der Formel 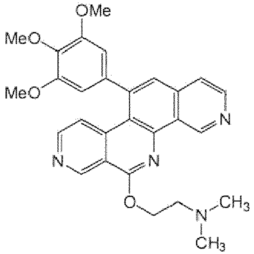 sowie deren Salze und/oder Solvate.

2. Verbindung nach Anspruch 1 **dadurch gekennzeichnet, dass** es sich um 6-(2-Dimethylaminoethoxy)-11-(3,4,5-trimethoxyphenyl)pyrido[3,4-c][1,9]-phenanthrolinhydrochlorid handelt.

3. Verfahren zur Herstellung einer Verbindung nach einem der Ansprüche 1 bis 2, umfassend die Schritte
i. Umsetzung von substituierten Benzaldehyden mit 4-Methylpyridin-3-carbonitril in aprotischen, dipolaren Lösungsmitteln in Gegenwart von Basen
ii. Dehydrierung des Produktes aus i zu einem vollständig konjugierten System, unter Einführung einer Doppelbindung in 11,1 2-Position
iii. Diazotierung und anschließende Hydrolyse des semicyclischen Amidins in eine Laktam-Struktur
iv. Veretherung der Verbindung aus iii im Sinne einer Mitsunobu-Reaktion

4. Arzneimittel, umfassend eine Verbindung nach einem der Ansprüche 1 bis 2.

5. Verbindung nach einem der Ansprüche 1 bis 2 zur Verwendung als Arzneimittel.

6. Verbindung nach einem der Ansprüche 1 bis 2 zur Verwendung in der Behandlung von Tumoren.

7. Arzneimittel nach Anspruch 4 zur Verwendung in der Behandlung von Tumoren.

8. Verbindung zur Verwendung nach Anspruch 6 oder Arzneimittel zur Verwendung nach Anspruch 7, wobei die Behandlung von Tumoren in der Hemmung der Topoisomerase besteht.

9. Verbindung nach einem der Ansprüche 1 bis 2 oder Arzneimittel nach Anspruch 4 zur Verwendung in der Behandlung von Leiden, die mit Topoisomeraschemmern behandelbar sind.

10. Verbindung oder Arzneimittel zur Verwendung nach Anspruch 8 oder 9, wobei Topoisomerase Typ I und Topoisomerase Typ II gleichzeitig gehemmt werden.

## Claims

1. A compound having the formula 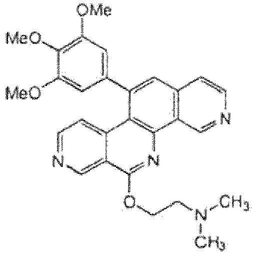 and salts and/or solvates thereof.

2. The compound according to claim 1, **characterized in that** the compound is 6-(2-dimethylaminoethoxy)-11-(3,4,5-trimethoxyphenyl)pyrido[3,4-c][1,9]-phenanthroline hydrochloride.

3. A method for producing a compound according to one of claims 1 to 2, comprising the steps of:
i. reacting substituted benzaldehydes with 4-methylpyridine-3-carbonitrile in dipolar aprotic solvents in the presence of bases,
ii. dehydrogenating the product from step i, resulting in a fully conjugated system, in so doing introducing a double bond in the 11, 12-position,
iii. diazotising and then hydrolysing the semicyclic amidine into a lactam structure,
iv. etherising the compound from step iii in a Mitsunobu-type reaction.

4. A medicinal product comprising a compound according to one of claims 1 to 2.

5. The compound according to one of claims 1 to 2 for use as a medicinal product.

6. The compound according to one of claims 1 to 2 for use in the treatment of tumors.

7. The medicinal product according to claim 4 for use in the treatment of tumors.

8. The compound for the use according to claim 6 or the medicinal product for the use according to claim 7, wherein,
the treatment of tumors involves the inhibition of topoisomerases.

9. The compound according to one of claims 1 to 2 or the medicinal product according to claim 4 for use in the treatment of diseases treatable using topoisomerase inhibitors.

10. The compound or medicinal product for the use according to claim 8 or 9, wherein type-I topoisomerases and type-II topoisomerases are inhibited simultaneously.

## Revendications

1. Composé de formule 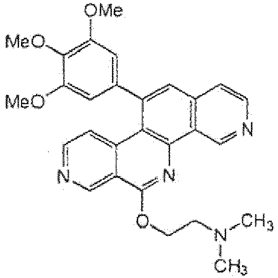 ainsi que ses sels et/ou solvates.

2. Composé selon la revendication 1 **caractérisé en ce qu'**il s'agit de chlorhydrate de 6-(2-diméthylaminoéthoxy)-11-(3,4,5-triméthoxyphényl)pyrido[3,4-c][1,9]-phénanthroline.

3. Procédé de fabrication d'un composé selon une des revendications 1 à 2, comprenant les étapes suivantes :
i. réaction de benzaldéhydes substitués avec du 4-méthylpyridine-3-carbonitrile dans des solvants dipolaires aprotiques en présence de bases
ii. déshydratation du produit de l'étape i jusqu'à obtenir un système entièrement conjugué, avec introduction d'une double liaison dans la position 11,12
iii. diazotation suivie de l'hydrolyse de l'amidine semi-cyclique dans une structure de lactame
iv. éthérification du composé de l'étape iii au sens d'une réaction de Mitsunobu

4. Médicament comprenant un composé selon une des revendications 1 à 2.

5. Composé selon une des revendications 1 à 2 destiné à être utilisé comme médicament.

6. Composé selon une des revendications 1 à 2 destiné à être utilisé dans le traitement de tumeurs.

7. Médicament selon la revendication 4 destiné à être utilisé dans le traitement de tumeurs.

8. Composé destiné à être utilisé selon la revendication 6 ou médicament destiné à être utilisé selon la revendication 7, le traitement de tumeurs consistant dans l'inhibition des topoisomérases.

9. Composé selon une des revendications 1 à 2 ou médicament selon la revendication 4 destiné à être utilisé dans le traitement de pathologies qui peuvent être traitées avec des inhibiteurs de topoisomérases.

10. Composé ou médicament destiné à être utilisé selon la revendication 8 ou 9, les topoisomérases de type I et les topoisomérases de type II étant bloqués en même temps.
